Europäisches Patentamt

(19) European Patent Office

Office européen des brevets

(11) Publication number: **0 214 761**
**B1**

# EUROPEAN PATENT SPECIFICATION

(45) Date of publication of the patent specification:
09.05.90

(51) Int. Cl.⁵: **C11D 3/386**

(21) Application number: **86306091.9**

(22) Date of filing: **06.08.86**

(54) An enzymatic detergent additive, a detergent, and a washing method.

(30) Priority: **07.08.85 DK 3588/85**

(43) Date of publication of application:
**18.03.87 Bulletin 87/12**

(45) Publication of the grant of the patent:
**09.05.90 Bulletin 90/19**

(84) Designated Contracting States:
**BE CH DE FR GB IT LI SE**

(56) References cited:
**FR-A- 2 121 170**
**GB-A- 1 442 418**

**PATENT ABSTRACTS OF JAPAN, vol. 6,
no. 139 (C-116)[1017], 28th July 1982; &
JP-A-57 63 087 (KOGYO GIJUTSUIN
(JAPAN)) 16-04-1982**

(73) Proprietor: **NOVO-NORDISK A/S, Novo Allé,
DK-2880 Bagsvaerd(DK)**

(72) Inventor: **Enomoto, Michiyo, 4-24-4, Higashi Kanamachi,
125 Katsushika-ku Tokyo(JP)**
Inventor: **Riisgaard, Steen, 37 Molledalen,
DK-2750 Ballerup(DK)**

(74) Representative: **Brown, John David et al, FORRESTER &
BOEHMERT Widenmayerstrasse 4/I,
D-8000 München 22(DE)**

## Description

The field comprising enzymatic additives in detergents has been rapidly growing during the last decades. Reference is made to e.g. the article "How Enzymes Got into Detergents", vol. 12, Developments is Industrial Microbiology, a publication of the Society for Industrial Microbiology, American Institute of Biological Sciences, Washington, D.C. 1971, by Claus Dambmann, Poul Holm, Villy Jensen and Mogens Hilmer Nielsen.

The most common enzymatic detergent additive is a proteolytic additive, but also lipolytic detergent additives are described, e.g. in US patent No. 4,011,169, column 4, line 65 to column 5, line 68, and British patent No. 1,293,613, page 2, lines 6 to 20.

Also, a comprehensive review article of lipases as detergent additives written by Hans Andree et al. is to be found in the Journal of Applied Biochemistry, 2, 218 - 229 (1980), entitled "Lipases as Detergent Components".

Among the known lipases used as detergent additives to the best of our knowledge the Fusarium oxysporum lipase has the best lipolytic characteristics, looked upon from a detergent application point of view, vide our European patent application with publication No. 0 130 064, especially the comparative table on page 27.

If the washing process is conducted at high temperature and high alkalinity, the majority of the fat containing dirt will be removed anyway. However, mainly due to the energy crisis and the fact that many modern-day fabrics have to be washed at low temperature, low temperature washing processes (around 60°C and below) are generally preferred, and at these low temperatures the known lipases are able to dissolve only a part of the fat containing dirt.

Hitherto the efficiency of lipolytio detergent additives usually has been measured by means of EMPA (Eidgenössische Materialprüfungs- und Versuchsanstalt, St. Gallen, Switzerland) swatches Nos. 101 (olive oil/cotton) and 102 (olive oil/wood) by adaptation of the procedure described in British patent No. 1,361,386, (especially pages 4 and 7) and US patent No. 3,723,250 (especially col. 15 - 19). In this way lipolytic cleaning efficiency can be expressed as the differential remittance value $\Delta$ R. However, it has been found that a more relevant measure for the lipolytic cleaning efficiency or the lipolytic detergency action is the determination of the amount of residual fat and residual fat decomposition products in the laundry after the lipolytic treatment comprising a lipolytic wash as compared to the amount of fat in the laundry before the lipolytic treatment, because this is a more direct and relevant effect of the lipolytic detergency action than the differential remittance value $\Delta$ R. By lipolytic detergency action we understand the detergency action caused by lipolytic activity. By use of this determination it has been found that even the best known detergent lipase exhibits a lipolytic detergency effect which is open to improvement.

Thus, a need exists for a lipolytic detergent additive which exhibits a considerably better lipolytic detergency efficiency at economically reasonable lipase activities in the washing solution.

Now, according to the first aspect of the invention, a lipolytic detergent additive has been found which exhibits a considerably better lipolytic detergency efficiency at economically reasonable lipase activities in the washing solution, this lipolytic detergent additive being characterized by the fact that the lipase is producible by means of a lipase producing strain of Pseudomonas cepacia.

It is described in Japanese patent application No. 135999 from 1980 that Pseudomonas cepacia is a lipase producer. However, it does not appear from this Japanese patent application that the Pseudomonas cepacia lipase, as described in Japanese patent application No. 135999 from 1980, is suited as an active component in an enzymatic detergent additive.

Also, in US patent No. 3,950,277 it is described in general terms that lipases from the genus Pseudomonas are suited as agents for removal of oily strains from fabrics, if used together with a special group of lipase activators. Also, it appears from this US patent that the lipases in consideration are well suited for pre-soaking of fabrics exclusively, whereas the enzymatic detergent additive according to the invention is well suited both as an ingredient in a presoaking detergent and in a main wash detergent.

Also, it is described in UK patent No. 1372034 that the lipase produced by means of Pseudomonas stutzeri ATCC 19154 is well suited an an enzymatic detergent additive in a detergent comprising specified further detergent constituents. It has been found, however, that the lipolytic detergency action of the enzymatic detergent additive according to the invention is superior to the lipolytic detergency action of the enzymatic detergent additive described in the UK patent.

It is to be understood that lipases which are immunologically identical to the lipases defined above are covered by the invention.

Also, it is to be understood that lipases deviating from the lipases defined above, but produced by genetic engineering on the basis of Pseudomonas cepacia, and with active centers identical to the active centers of the lipases producible by means of a lipase producing strain of Pseudomonas cepacia, are also covered by the invention.

Some strains belonging to Pseudomonas cepacia are poor lipase producers. For the purposes of this invention, a lipase producing strain of Pseudomonas cepacia is defined as a strain which produces more that 10 LU/ml or more than 2 $NLU_{8.5}$/ml (the LU and $NLU_{8.5}$ being the lipase units defined later in this specification) in a suitable medium, e.g. a medium designated LIP7.

The medium LIP7 intended for shaking flasks is prepared with the following ingredients in grams per litre:

```
PHARMAMEDIA              20

corn steep liquor        40

glucose                  10

soybean oil      _       40

pH adjusted to 7.4 before autoclaving

final pH 7.0
```

The composition of PHARMAMEDIA is described in Traders' Guide to Fermentation Media Formulation, 1980, Traders Oil Mill Co., p. 50 - 51.

Sterilization took place at around 130°C for around 40 minutes. A 500 ml Erlenmeyer flask with 100 ml of substrate was inoculated with cells from an agar slant previously inoculated with the strain to be tested for lipase production. The flasks were shaken at 200 - 250 rpm and at around 26°C for 4 days, whereafter the lipase yield was determined.

Thus, it has surprisingly been found that the enzymatic detergent additive according to the invention exhibits an improved lipolytic cleaning efficiency which will appear from documentation presented later in this specification.

In this specification two (or three) different lipase activity units are used, i.e. the Lipase Unit (LU) described in AF 95/5-GB, and the NOVO Lipase Unit (NLU) described in AF 182/3-GB, both AF documents being available on request from NOVO INDUSTRI A/S, Novo Alle, 2880 Bagsvaerd, Denmark, and furthermore a modified Novo Lipase Unit, differing only from the Novo Lipase Unit (determined at pH 7.0 or closely to pH 7.0) by the fact that the activity determination is carried out at pH 8.5, this modified Novo Lipase Unit for the sake of brevity being designated $NLU_{8.5}$. The LU method is a quick and simple activity determination method based on a substrate fat with a short chain, i.e. tributyrin, and thus not representative for soiling fats on textiles; the NLU method is a more elaborate activity determination method based on olive oil, which is a mixture of triglycerides containing longer chain fatty acids, considered more representative for soiling fats on textiles.

In a specially preferred embodiment of the enzymatic detergent additive according to the invention the strain is Pseudomonas cepacia DSM 3333 to 3337 inclusive, or 3401, preferably 3335 or 3401. It has been found that these strains give rise to a relatively high yield of lipase.

In a specially preferred embodiment of the enzymatic detergent additive according to the invention, the additive is provided as a non-dusting granulate. Granulates can be produced in several different ways. Reference can be made to GB patent No. 1,362,365 which describes the production of enzyme containing granulates used as detergent additives by means of an apparatus comprising an extruder and a spheronizer (sold as MARUMERIZER®), and to US patent No. 4,106,991 which describes the production of enzyme containing granulates used as detergent additives by means of a drum granulator.

In a specially preferred embodiment of the enzymatic detergent additive according to the invention the additive is provided as a liquid. Liquid detergents exhibit a growing popularity due to the ease of application.

In a specially preferred embodiment of the enzymatic detergent additive according to the invention, the additive is provided as a liquid with an enzyme stabilizer. The stabilizer can be propylene glycol or other agents known as stabilizers for enzyme solutions.

In a specially preferred embodiment of the enzymatic detergent additive according to the invention, the lipase activity is above about 2500 NLU/g of additive. In this manner, a convenient lipase activity is generated in the washing solution when the detergent additive is added to the detergent in an amount of 0.01 to 50.0 g/100 g of detergent, and when the detergent is added to the washing solution in an amount of 1 - 20 g of detergent/l of washing solution.

In a specially preferred embodiment of the enzymatic detergent additive according to the invention, the additive contains a proteolytic enzyme besides the lipase. As such proteolytic enzyme the proteolytic enzyme ALCALASE® from NOVO INDUSTRI A/S, manufactured microbially by means of Bacillus liceniformis, can be used. The mixed enzymatic additive can be prepared either by mixing a previously prepared granulate of proteinase with a previously prepared granulate of lipase, or by mixing a concentrate of proteinase with a concentrate of lipase and then introducing this mixture into a granulating device, together with the usual granulating aids.

In a specially preferred embodiment of the enzymatic detergent additive according to the invention, the proteolytic activity is between about 0.5 and about 3.0 Anson Units/g of additive. In this manner, a convenient proteolytic activity is generated in the washing solution when the detergent additive is added to the detergent in an amount of 0.2 - 2 g/100 g of detergent, and when the detergent is added to the washing solution in an amount of 1 - 20 g of detergent/l of washing solution.

The second aspect of the invention comprises a detergent comprising an enzymatic detergent additive, the active component of which is a microbially produced lipase, wherein the enzymatic detergent additive is the enzymatic detergent additive according to the invention.

In a specially preferred embodiment of the detergent according to the invention, the detergent contains the enzymatic detergent additive according to the invention in an amount of between 0.01 and 50.0% w/w, preferably between 0.1 and 2% w/w. In this manner, a reasonable balance between enzyme action and the action of the other detergent ingredients is generated.

The third aspect of the invention comprises a washing process in which the detergent used is the detergent according to the invention and in which the pH is between 7 and 11.

In a specially preferred embodiment of the washing process according to the invention, the washing solution contains the detergent according to the invention in an amount of between 1 and 20 g/l of washing solution. In this manner, a convenient lipase activity is generated in the washing solution, i.e. typically between 1,000 and 5,000 NLU/l of washing solution.

The invention will be illustrated by the following examples, of which examples 1 - 7 are production examples, and examples 8 - 13 are washing examples.

Example 1

A culture of Pseudomonas cepacia DSM 3401 on an agar slant was transferred to five 500 ml shaking flasks, each with 100 ml of Boullion-3 medium, and shaken at 30°C for 1 day (200 rpm, amplitude 2.5 cm).

The composition of Boullion-3 was as follows:

| constitutent | concentration, g/l |
|---|---|
| Peptone | 6 |
| Trypsin digested casein | 4 |
| Yeast extract | 3 |
| Meat extract | 1.5 |
| Glucose | 1 |

The medium was autoclaved at 121°C for 40 minutes.

The culture broth of these Boullion-3 shake flasks were used as a seed culture for inoculating two hundred 500 ml shake flasks, each with 200 ml PL-1 medium.

The composition of the PL-1 medium was as follows:

| constitutent | concentration, g/l |
|---|---|
| Peptone | 10 |
| Tween-80 | 12 |
| $MgSO_4 \cdot 7H_2$ | 2 |
| $CaCl_2 \cdot 2H_2O$ | 0.1 |
| pH before autoclaving | 6.0 |

The medium was autoclaved at 121°C. for 40 minutes.

Each PL-1 shake flask was inoculated with 0.5 - 2 ml of Boullion-3 culture broth, and shaken with 200 rpm (amplitude 2.5 cm) at 30°C for 5 days. The culture broth from the shake flasks were pooled at harvest, totalling 39.5 l with an enzyme yield of 53 LU/ml.

The culture broth was centrifuged for 35 minutes at 4100 g by means of a Beckman Model J-6 centrifuge. The supernatant was concentrated by ultrafiltration (washed with approximately 1 volume of water) to 1.4 l by a Pellicon ultrafiltration apparatus from Millipore with a 10000 MW cut off filter sheet. The concentrate was freeze dried, and the yield was 56.2 g of powder with a enzyme activity of 21.500 LU/g.

In the following production examples 2 - 6 100 ml of medium in a 500 ml shake flask was inoculated with cultures originating from specified strains of Pseudomonas cepacia on agar slants incubated at around 30°C for around 3 days. Example 7 is a comparison example representing Fusarium oxysporum lipase, obtained as described in example 23 in European patent application with publication No. 0 130 064, and intended for use in example 13, which is a comparative washing example. The medium in examples 2 - 6 is LIP7 (described previously).

The shake flasks were incubated for 4 days at around 26°C and at 200 - 250 rpm. The lipase activity of the broth (NLU$_{8.5}$/ml) was measured, and the broth was directly used as a lipase source in a later described washing example.

| Production example No. | Strain DSM No. | Lipase activity of | | Corresponding washing example No. |
|---|---|---|---|---|
| | | broth NLU$_{8.5}$/ml | powder NLU$_{8.5}$/g | |
| 2 | 3333 | 13.0 | | 8 |
| 3 | 3334 | 7.7 | | 9 |
| 4 | 3337 | 7.1 | | 10 |
| 5 | 3335 | 10.4 | | 11 |
| 6 | 3336 | 4.1 | | 12 |
| 7 | - | - | 28300 | 13 |

The washing examples 8 - 13 were carried out as follows.

Cotton swatches (obtained from the Japan Oil Chemists' Society and designated No. 6) measuring 7.5 x 5.0 cm and weighing 0.52 g were treated with 0.5 ml of 3% beef tallow solution in chloroform on each side.

The swatches were cut into 8 pieces of approximately the same size. These 8 pieces were transferred to a small reaction vessel containing 10 ml of enzyme solution with specified lipase activity (NLU$_{8.5}$/ml) and with pH 8.5, controlled by means of an approximately 0.2 M buffer of TRIS/HCl (TRIS = 2-amino-2-hydroxymethyl-1,3-propanediol). The reaction mixture was incubated for 1 hour at 40°C with shaking. Then 40 µl of 10% LAS in 0.2 M borate buffer (pH 10) was added to the reaction mixture. Now the reaction mixture was incubated for 15 minutes at 40°C with shaking. 100 µl of the washing solution was withdrawn for the assay of free fatty acids.

The assay of free fatty acids was carried out colorimetrically in accordance with the method described in the brochure Code 279-75401 Nefa C-test Wako obtainable from Wako Chemicals, GmbH, Nissan Strasse 2-4040, Neussl, West Germany, except that a 100 µl sample was used instead of a 50 µl sample, reagent samples A and B were 0.5 and 1 ml instead of 1 and 2 ml, respectively and that the assay was carried out at 40°C instead of 37°C. A cuvette with a path length of 1 cm was used for the spectrophotometric measurement. The spectrophotometric reading at 550 nm $A_1$ for the washing solution being corrected for the spectrophotometric reading at 550 nm $A_0$ for a blank (a 100 µl enzyme sample taken out from the reaction vessel before incubation) is the $\Delta A$ value ($\Delta A = A_1 - A_0$) representing the amount of free fatty acids in the washing solution liberated from the swatches. Thus, $\Delta A$ is an indication of the lipolytic washing efficiency.

The following washing examples 8 - 12 illustrate the invention. Example 13 is the comparative washing example, performed with Fusarium oxysporum lipase.

| Example No. | Lipase origi- nating from example No. | Lipase acitivty in washing solu- tion, $NLU_{8.5}$/ml | $\Delta A$ |
|---|---|---|---|
| 8 | 2 | 2.6 | 0.48 |
| 9 | 3 | 1.6 | 0.53 |
| 10 | 4 | 2.8 | 0.41 |
| 11 | 5 | 2.1 | 0.59 |
| 12 | 6 | 2.1 | 0.56 |
| 13 | 7 | 2.8 | 0.21 |

It clearly appears from the above table that the enzymatic detergent additive according to the invention is superior to the Fusarium oxysporum lipase, in terms of free fatty acid released from the swatches in the washing solution, at comparable values of lipase activity expressed as $NLU_{8.5}$/ml.

Deposits DSM 3333 to 3337 and 3401 were all made at the Deutsche Sammlung für Mikroorganismen under the terms and conditions of the Budapest Treaty. Deposits DSM 3333 to 3336 were made on 28 May 1985, DSM 3337 was made on 10 June 1985 and DSM 3401 was made on 22 July 1985.

**Claims**

1. Enzymatic detergent additive the active component of which is a microbially produced lipase, characterized in that the lipase is producible by means of a lipase producing strain of Pseudomonas cepacia.

2. Enzymatic detergent additive according to claim 1, wherein the strain is Pseudomonas cepacia DSM 3333 to 3337 inclusive, or 3401, preferably DSM 3335 or 3401.

3. Enzymatic detergent additive according to claims 1 to 2, wherein the additive is provided as a non-dusting granulate.

4. Enzymatic detergent additive according to claims 1 or 2, wherein the additive is provided as a liquid.

5. Enzymatic detergent additive according to claim 4, wherein the liquid contains an enzyme stabilizer.

6. Enzymatic detergent additive according to claims 1 to 5, wherein the lipase activity is above about 2500 NLU/g of additive.

7. Enzymatic detergent additive according to claims 1 to 6, wherein the additive besides the lipase contains a proteolytic enzyme.

8. Enzymatic detergent additive according to claim 7, wherein the proteolytic activity is between about 0.5 and about 3.0 Anson units/g of additive.

9. Detergent comprising an enzymatic additive, the active component of which is a microbially produced lipase, characterized in that the enzymatic detergent additive is the enzymatic additive according to claims 1 to 8.

10. Detergent according to claim 9, wherein the detergent contains the enzymatic detergent additive according to claim 1 to 8 in an amount of between 0.01 and 50.0% w/w, preferably between 0.1 and 2% w/w.

11. Washing process using a detergent which is the detergent according to claims 9 - 10 and wherein the pH is between 7 and 11.

12. Washing process according to claim 11, wherein the washing solution contains the detergent according to claims 9 to 10 in an amount of between 1 and 20 g per litre of washing solution.

**Patentansprüche**

1. Enzymatischer Reinigungsmittelzusatz, dessen wirksamer Bestandteil eine mikrobiell hergestellte Lipase ist, dadurch gekennzeichnet, daß die Lipase mit Hilfe eines lipaseerzeugenden Stamms von Pseudomonas cepacia herstellbar ist.

2. Enzymatischer Reinigungsmittelzusatz nach Anspruch 1, dadurch gekennzeichnet, daß der Stamm Pseudomonas cepacia DSM 3333 bis 3337 einschließlich, oder 3401, vorzugsweise DSM 3335 oder 3401 ist.

3. Enzymatischer Reinigungsmittelzusatz nach den Ansprüchen 1 bis 2, dadurch gekennzeichnet, daß der Zusatz als ein nicht-staubendes Granulat zur Verfügung gestellt wird.

6

4. Enzymatischer Reinigungsmittelzusatz nach den Ansprüchen 1 oder 2, dadurch gekennzeichnet, daß der Zusatz als eine Flüssigkeit zur Verfügung steht.

5. Enzymatischer Reinigungsmittelzusatz nach Anspruch 4, dadurch gekennzeichnet, daß die Flüssigkeit einen Enzymstabilisator enthält.

6. Enzymatischer Reinigungsmittelzusatz nach den Ansprüchen 1 bis 5, dadurch gekennzeichnet, daß die Lipaseaktivität über etwa 2500 NLU/g des Zusatzes liegt.

7. Enzymatischer Reinigungsmittelzusatz nach den Ansprüchen 1 bis 6, dadurch gekennzeichnet, daß der Zusatz neben der Lipase ein proteolytisches Enzym enthält.

8. Enzymatischer Reinigungsmittelzusatz nach Anspruch 7, dadurch gekennzeichnet, daß die proteolytische Aktivität zwischen etwa 0,5 und etwa 3,0 Anson-Einheiten/g des Zusatzes liegt.

9. Reinigungsmittel mit einem enzymatischen Zusatz, dessen wirksamer Bestandteil eine mikrobiell hergestellte Lipase ist, dadurch gekennzeichnet, daß der enzymatische Reinigungsmittelzusatz der enzymatische Zusatz nach den Ansprüchen 1 bis 8 ist.

10. Reinigungsmittel nach Anspruch 9, dadurch gekennzeichnet, daß das Reinigungsmittel den enzymatischen Reinigungsmittelzusatz gemäß den Ansprüchen 1 bis 8 in einer Menge von zwischen 0,01 und 50,0 Gew.-%, vorzugsweise zwischen 0,1 und 2,0 Gew.-%, enthält.

11. Waschverfahren unter Verwendung eines Reinigungsmittels, welches das Reinigungsmittel gemäß den Ansprüchen 9 bis 10 ist und wobei der pH zwischen 7 und 11 liegt.

12. Waschverfahren nach Anspruch 11, dadurch gekennzeichnet, daß die Waschlösung das Reinigungsmittel gemäß den Ansprüchen 9 bis 10 in einer Menge von zwischen 1 und 20 g pro Liter Waschlösung enthält.

**Revendications**

1. Additif enzymatique pour détergent dont le constituant actif est une lipase produite microbiologiquement, caractérisé en ce que la lipase peut être produite au moyen d'une souche productrice de lipase de Pseudomonas cepacia.

2. Additif enzymatique pour détergent suivant la revendication 1, dans lequel la souche est Pseudomonas cepacia DSM 3333 à 3337 compris ou 3401, de préférence DSM 3335 ou 3401.

3. Additif enzymatique pour détergent suivant les revendications 1 à 2, dans lequel l'additif est apporté sous la forme d'un granulé ne produisant pas de poussière.

4. Additif enzymatique pour détergent suivant les revendications 1 ou 2, dans lequel l'additif est apporté sous forme d'un liquide.

5. Additif enzymatique pour détergent suivant la revendication 4, dans lequel le liquide contient un stabilisant de l'enzyme.

6. Additif enzymatique pour détergent suivant les revendications 1 à 5, dans lequel l'activité de lipase est supérieure à environ 2500 NLU/g d'additif.

7. Additif enzymatique pour détergent suivant les revendications 1 à 6, dans lequel l'additif, outre la lipase, contient une enzyme protéolytique.

8. Additif enzymatique pour détergent suivant la revendication 7, dans lequel l'activité protéolytique est comprise entre environ 0,5 et environ 3,5 unités Anson/g d'additif.

9. Détergent comprenant un additif enzymatique, dont le constituant actif est une lipase produite microbiologiquement, caractérisé en ce que l'additif pour détergent est l'additif enzymatique suivant les revendications 1 à 8.

10. Détergent suivant la revendication 9, dans lequel le détergent contient l'additif enzymatique pour détergent suivant les revendications 1 à 8 dans une quantité comprise entre 0,01 et 50,0% P/P, de préférence entre 0,1 et 2% P/P.

11. Procédé de lavage utilisant un détergent qui est le détergent suivant les revendications 9–10 et dans lequel le pH est compris entre 7 et 11.

12. Procédé de lavage suivant la revendication 11, dans lequel la solution de lavage contient le détergent suivant les revendications 9 à 10 dans une quantité comprise entre 1 et 20 g par litre de solution de lavage.